# EUROPÄISCHE PATENTSCHRIFT

(11) **EP 1 904 121 B1**
(45) Veröffentlichungstag und Bekanntmachung des Hinweises auf die Patenterteilung: **12.03.2014**
(21) Anmeldenummer: 06743025.6
(22) Anmeldetag: 23.05.2006
(51) Int. Cl.: A61M 1/28, A61J 1/00, A61M 39/08

(54) **MEDIZINISCHE VORRICHTUNG**
MEDICAL DEVICE
DISPOSITIF MEDICAL

(30) Priorität: 23.05.2005 DE 102005024151
(43) Veröffentlichungstag der Anmeldung: 02.04.2008
(73) Patentinhaber: Schmidtlein, Martin, 66113 Saarbrücken (DE); Nicola, Thomas, 57350 Spicheren (FR)
(72) Erfinder: Schmidtlein, Martin, 66113 Saarbrücken (DE); Nicola, Thomas, 57350 Spicheren (FR)
(74) Vertreter: Isenbruck, Günter
(86) Internationale Anmeldenummer: PCT/EP2006/004881
(87) Internationale Veröffentlichungsnummer: WO 2006/125603

(56) Entgegenhaltungen:
- EP-A- 0 224 780
- EP-A- 0 739 713
- EP-A- 0 776 649

## Beschreibung

Die Erfindung betrifft eine medizinische Vorrichtung zum Entnehmen einer Flüssigkeit aus einem Behälter oder zu dessen Befüllung, ein mit dieser Vorrichtung durchzuführendes Verfahren sowie die Verwendung der Vorrichtung zur Entnahme von Flüssigkeiten aus einem Behälter im medizinischen Bereich, insbesondere zur Durchführung von Peritonealdialysen.

Im Bereich der Medizintechnik ergibt sich häufig das Problem, eine Flüssigkeit, vorzugsweise unter sterilen Bedingungen aus einem Behälter in einen anderen oder auch den menschlichen Körper zu leiten, beispielsweise im Rahmen einer Infusion.

Gegenwärtig erfolgt der Transport von Flüssigkeiten im medizinischen Bereich überwiegend durch spezielle Schläuche. Solche Schläuche sind kostenintensiv in der Herstellung, was bei der weltweit angespannten Finanzlage der Gesundheitssysteme eine immer größere Rolle spielt. Weiterhin neigen die genannten Schläuche häufig zum Knicken unter Verschluss des Schlauches ("Kinking"). In der EP-A 0 224 780 ist eine Vorrichtung zum Spülen des Darms, die interoperativ verwendbar ist, mit einer Spülsonde zum Einleiten der Spülflüssigkeit und einer Ableitungsvorrichtung beschrieben.

Es besteht daher ein Bedarf für eine Vorrichtung zur Entnahme von Flüssigkeiten im medizinischen Bereich, welche die Nachteile bekannter Systeme überwindet, d.h. insbesondere kostengünstig herzustellen ist und nicht zum Kinking neigt.

Aufgabe der Erfindung ist die Bereitstellung einer solchen Vorrichtung.

Gegenstand der Erfindung ist daher eine medizinische Vorrichtung, umfassend
(a) einen zur Aufnahme von Flüssigkeiten geeigneten Behälter,
(b) damit verbunden einen Folienschlauch mit einer Konnektionsstelle und
(c) gegebenenfalls einen oder mehrere weitere, mit dem Folienschlauch verbundene, zur Aufnahme von Flüssigkeiten geeignete Behälter,
dadurch gekennzeichnet dass der Verbindungs-Folienschlauch durch Peelnähte und/oder Ventile in kammern unterteilt ist.

Die erfindungsgemäße Vorrichtung ist kostengünstig herzustellen und vermindert das Problem des Kinking. Sie kann in sämtlichen Kliniken, insbesondere auch in ärmeren Ländern, für den Einsatzfall ohne größere Kosten auf Lager gehalten werden.

Bei den Behältern (a) und gegebenenfalls (c) handelt es sich vorzugsweise um Beutel, insbesondere um Schlauchbeutel.

Im Rahmen der Erfindung wird unter dem Begriff "Schlauchfolie", insbesondere hinsichtlich einer Abgrenzung gegenüber dem Begriff eines "Schlauches", verstanden, dass eine Schlauchfolie - im Gegensatz zum Schlauch - nur Rückstellkräfte aufweist, die wesentlich geringer als die Gewichtskraft sind. Dies bedeutet, dass der Folienschlauch ohne den Gegendruck durch eine Füllung in sich zusammenfällt. Demgegenüber weist ein Schlauch eine Rückstellkraft auf, welche nach einem Zusammendrücken des Schlauches dafür sorgt, dass der Schlauch wieder auf den im Wesentlichen ursprünglichen Durchmesser zurückkehrt. Darüber hinaus ist die Wandstärke eines Folienschlauches geringer als bei einem Schlauch. Im Rahmen der vorliegenden Erfindung weist der erfindungsgemäß zu verwendende Folienschlauch eine Wandstärke von vorzugsweise 50 bis 300 µm, besonders bevorzugt 50 bis 150 µm, insbesondere 50 bis 120 µm, auf.

Bei dem Folienschlauch handelt es sich somit um ein kollabierbares Verbindungssystem.

Der Folienschlauch ist vorzugsweise so beschaffen, dass die Innenseite bei einer Wärmebehandlung mit für den medizinischen Bereich typischen Temperaturen, insbesondere einer Sterilisierungstemperatur von 121 °C, nicht verklebt.

Die Innenfläche des Folienschlauchs ist daher im Allgemeinen strukturiert, bevorzugt aufgerauht. Eine Strukturierung kann beispielsweise durch Prägen erfolgen, bevorzugt erfolgt ein Aufrauhen beim Extrudieren durch Wahl einer geeigneten Düsenform.

Die Rauhigkeit lässt sich beispielsweise mikroskopisch bestimmen. Bevorzugt sind Strukturen mit einer Rauhtiefe R_{z} von 0,05 bis 10 µm, vorzugsweise 0,1 bis 3 µm, insbesondere 0,1 bis 2 µm. Die Rauhtiefe R_{z} ist dabei gemäß ISO/DIS 4287/1 definiert.

Der Folienschlauch ist durch Peelnähte und/oder Ventile in Kammern unterteilt.

In einer weiteren bevorzugten Ausführungsform sind je nach gewünschtem Verwendungszweck die Verbindungen verschließbar. Zum Verschließen eignen sich dabei beispielsweise trennbare Peelnähte, Ventile oder Klammern. Die Funktionen Auslauf und Einlauf werden daher vorzugsweise über trennbare Peelnähte, Ventile oder Klammern gesteuert.

Der Verbund zwischen dem zur Aufnahme von Flüssigkeiten geeigneten Behälter und dem Folienschlauch kann auf jede geeignete Weise erfolgen:

Beispielhaft sei hierfür das Verschweißen des Verbindungs-Folienschlauches mit dem Behälter genannt, wobei die Außenseite des Verbindungs-Folienschlauches mit der Innenseite des Behälters oder aber die Innenseite des Verbindungs-Folienschlauchs mit dem Behälter verschweißt ist. Der Verbindungs-Folienschlauch kann dabei über Peelnähte oder Ventile abgetrennt sein.

Alternativ kann der Behälter auch über ein Portelement mit dem Folienschlauch verbunden werden. In diesem Fall kann das Portelement mit dem Beutel und dem Verbindungs-Folienschlauch als Zwischenglied verschweißt sein.

Unter einem Portelement wird im Rahmen der Erfindung beispielsweise ein Sattelelement verstanden. Entsprechende Sattelelemente sind in der WO 90/06262 beschrieben.

Damit die Flüssigkeit nicht unmittelbar nach dem Anschluss der Vorrichtung abfließt, kann die Verbindung zwischen einem weiteren Behälter und dem Folienschlauch zunächst verschlossen sein. Hierfür eignet sich neben den bereits genannten Verschlussarten auch eine Aufreißversiegelung, wie eine Peelnaht.

Der Verbindungs-Folienschlauch besitzt in einer bevorzugten Ausführungsform eine zweite Konnektionsstelle, beispielsweise zum Peritoneum eines Patienten. Diese zweite Verbindung wird vorzugsweise mit einem Katheder verbunden, der beispielsweise in das Peritoneum des Patienten führt. Bei dem Katheter handelt es sich zum Beispiel um einen Dauerkatheter. Die Verbindung des erfindungsgemäß vorgesehenen Folienschlauches für den Katheder ist vorzugsweise als ein Y-Teil oder ein Sattel ausgebildet, der auf den Folienschlauch aufgebracht ist. Der Sattel kann dabei beispielsweise wie in der WO 90/06262 beschrieben ausgebildet sein.

Ein auf dem Verbindungs-Folienschlauch zum Anschluss an einen Katheter angebrachtes Y-Teil bzw. der auf dem Verbindungs-Folienschlauch zum Anschluss an den Katheter angebrachte Sattel sollte vorzugsweise folgende Strömungssequenzen ermöglichen:
(1) Flüssigkeit, beispielsweise eine Drainagelösung, sollte beispielsweise aus der Bauchhöhle eines Patienten in einen gegebenenfalls vorhandenen weiteren Behälter, beispielsweise einen leeren Drainagebeutel fließen können.
(2) Die Flüssigkeit, beispielsweise eine Dialysatflüssigkeit, sollte durch den Folienschlauch, gegebenenfalls den Sattel bzw. gegebenenfalls das Y-Teil in den gegebenenfalls vorhandenen Behälter, beispielsweise einen Drainagebeutel, hindurchgespült werden, um die Leitung zu reinigen.
(3) Die Flüssigkeit, beispielsweise eine Dialysatflüssigkeit, sollte in die Bauchhöhle eines Patienten eingeführt werden.

Der Sattel bzw. das Y-Teil sollte darüber hinaus vorzugsweise dafür sorgen, dass keine Flüssigkeit in unnötiger Weise verloren geht, beispielsweise nachdem die Vorrichtung von einem Patienten getrennt wurde.

Der Verbindungs-Folienschlauch besitzt darüber hinaus gegebenenfalls eine dritte Verbindung, die an einen weiteren Behälter angebunden sein kann.

Der Verbindungs-Folienschlauch ist mit diesem weiteren Behälter, beispielsweise dem Drainagebeutel, vorzugsweise unter Einhaltung der Sterilitätsbedingungen verbunden.

Der Verbund zwischen dem Behälter und dem Verbindungs-Folienschlauch kann dabei auf jede geeignete Weise erfolgen:
Beispielhaft sei hierfür das Verschweißen des Folienschlauches mit dem Behälter genannt, wobei - je nach verwendetem Material des Behälters und des Folienschlauches - der Verbindungs-Folienschlauch entweder mit seiner Außenseite oder seiner Innenseite des Behälters verschweißt ist. Zur Trennung zum Lösungszweig ist vorzugsweise eine Peelnaht, ein Ventil oder eine Klemme vorgesehen.

Alternativ kann der Behälter, beispielsweise Drainagebeutel, auch über ein Portelement, wie beispielsweise ein Sattel, mit dem Folienschlauch verbunden werden. In diesem Fall kann das Portelement mit dem Behälter und dem Verbindungs-Folienschlauch als Zwischenglied verschweißt sein.

Bei einer bevorzugten Ausführungsform der Erfindung sind der Behälter zur Aufnahme einer Flüssigkeit und gegebenenfalls vorhandener weiterer Behälter aus biegsamen Kunststoffmaterialen hergestellt. Dabei handelt es sich bevorzugt um coextrudierte Mehrschichtfolien aus Kunststoffen, die auf Polyolefinen basieren.

In einer bevorzugten Ausführungsform der Erfindung sind die für die erfindungsgemäße Vorrichtung verwendeten Behälter frei von PVC.

In einer weiteren bevorzugten Ausführungsform der vorliegenden Erfindung sind die für die erfindungsgemäße Vorrichtung verwendeten Behälter frei von Weichmachern.

In einer weiteren bevorzugten Ausführungsform der vorliegenden Erfindung sind die für die erfindungsgemäße Vorrichtung verwendeten Behälter frei von PVC und Weichmachern.

Der mit dem Behälter verbundene Verbindungs-Folienschlauch weist eine oder, vorzugsweise, mehrere Schichten auf. Die Herstellung des Mehrschichtfolienschlauches kann auf dem Fachmann an sich bekannte Art und Weise erfolgen. Geeignet hierfür ist beispielsweise das in der Patentanmeldung DE-A 103 61 851 beschriebene Verfahren.

Geeignete Mehrschichtfolien weisen vorzugsweise bis zu 7 Folienschichten auf. Besonders bevorzugt sind Mehrschichtfolien mit 2 bis 5 Schichten, insbesondere 3 Schichten.

Für die jeweiligen Schichten geeignete Materialien sind beispielsweise Mischungen aus modifiziertem Polypropylen. Dabei kann das Polypropylen in Mischung mit synthetischen Kautschuken, beispielsweise SEBS- (Styrol/Ethylen-Butylen/Styrol), SEPS-(Styrol/Ethylen-Propylen/Styrol) und SIS-Kautschuken (Styrol/Isopren/Styrol), vorliegen. Die SEBS und SEPS Kautschuke sind gegebenenfalls mit einem Öl modifiziert. Auch Copolymere aus Polyethylen und Polypropylen sind bevorzugt.

Die Phasenmatrix-Polymere enthalten im Falle von Dreischichtfolien neben der Polypropylen-Matrix in der Innenschicht im Allgemeinen 10 bis 40 Gew.-%, in der Mittelschicht 20 bis 60 Gew.-% und in der Außenschicht 0 bis 40 Gew.-% an einer Phase, vorzugsweise einer Blockcopolymerphase.

Durch die Wahl geeigneter Mischungen der vorgenannten Kunststoffe in den jeweiligen Schichten können Folienmaterialien mit unterschiedlichen Eigenschaften erhalten werden.

In einer bevorzugten Ausführungsform der vorliegenden Erfindung ist der Verbindungs-Folienschlauch aus mindestens zwei Schichten ausgebildet, wobei die Schmelztemperatur der Innen- und Außenschicht je nach Verwendung des Verbindungs-Folienschlauches eingestellt wird.

Bei dem Material der Außenschicht handelt es sich in einer bevorzugten Ausführungsform um ein Material, dass als Hauptbestandteil ein Homopolymer aus Polypropylen umfasst, dessen Schmelzpunkt vorzugsweise ungefähr bei 161 °C beträgt.

In einer weiteren bevorzugten Ausführungsform ist die Außenschicht eine Siegelschicht, die ein Homo- oder Copolymer auf Polypropylenbasis mit einem Schmelzpunkt von ungefähr 125 bis 150 °C enthält.

Wenn eine dreischichtige Folienschlauch-Struktur vorliegt, so fungiert als Mittelschicht vorzugsweise ein Polypropylen-Copolymer. Alternativ kann die Mittelschicht auch ein Random-heterophasiges Copolymer aus Polypropylen mit vorzugsweise einer der oben genannten Phasen umfassen, das dem Folieschlauch zur Erhöhung der Schlagzähmodifizierung zugesetzt wird. Die Mittelschicht weist vorzugsweise einen Schmelzpunkt von 121 bis 150 °C, besonders bevorzugt 124 bis 135 °C auf.

Die Innenschicht des erfindungsgemäßen Verbindungs-Folienschlauches ist in einer bevorzugten Ausführungsform wie die Mittelschicht ausgebildet, wobei der Innenschicht insgesamt weniger Schlagzähmodifizierer zugesetzt werden kann, so dass sich bessere Schweißeigenschaften des resultierenden Folienschlauches ergeben. Vorzugsweise ist die Innenschicht aus einem Material aufgebaut, das als Hauptbestandteil ein Polypropylen-Polymer umfasst, dessen Schmelzpunkt größer als 135 °C ist.

In einer weiteren bevorzugten Ausführungsform ist die Innenschicht eine temperaturresistente Schicht, enthaltend ein Homo- oder Copolymer auf Polypropylenbasis mit einem Schmelzpunkt von 125 bis 161 °C.

Anstelle von Polypropylen kann als Basismaterial auch Polyethylen verwendet werden, wobei die Materialien dann eine entsprechend niedrigere Schmelztemperatur aufweisen.

In einer bevorzugten Ausführungsform der vorliegenden Erfindung sind die für die erfindungsgemäße Vorrichtung verwendeten Verbindungs-Folienschläuche frei von PVC.

In einer weiteren bevorzugten Ausführungsform der vorliegenden Erfindung sind die für die erfindungsgemäße Vorrichtung verwendeten Verbindungs-Folienschläuche frei von Weichmachern.

In einer weiteren bevorzugten Ausführungsform der vorliegenden Erfindung sind die für die erfindungsgemäße Vorrichtung verwendeten Verbindungs-Folienschläuche frei von PVC und Weichmachern.

Der Behälter (a) sowie weitere Behälter (c), falls vorhanden, sowie der Verbindungs-Folienschlauch können nach Verwendung vorzugsweise gemeinsam entsorgt werden.

Die erfindungsgemäße Vorrichtung wird vorzugsweise wie folgt hergestellt:

Die Vorrichtungskomponenten liegen als Schlauchfolie oder als randbeschnittene Schlauchfolie in Form von Rollenware vor. Portelemente, zum Beispiel Sattelstücke, liegen als Einzelteile vor.

Die Beutelkontur(en) wird/werden mittels Schweißvorrichtung durch Erhitzen unter Druck unlösbar verschweißt. Dann wird der Verbindungs-Folienschlauch oder das Portelement in die Beutelkontur eingeführt und ebenfalls mit der Beutelkontur unlösbar verschweißt.

Wenn ein Portelement verwendet wird, so wird dieses dann mit dem Verbindungs-Folienschlauch durch Verschweißen unlösbar verbunden.

Peelnähte (lösbare Verschweißungen) und/oder Ventile werden als Kammertrennung oder Funktionsvorgabe eingeschweißt.

Eine Differenzierung bzw. Steuerung, ob eine lösbare oder unlösbare Verschweißung erzeugt wird, kann durch Variation von Schweißtemperatur, Schweißdruck und/oder Schweißzeit erfolgen.

Die Konnektionsstelle, beispielsweise ein Patientenanschluss, kann in oder auf den Verbindungs-Folienschlauch unlösbar verschweißt angebracht werden.

Die erfindungsgemäße Vorrichtung eignet sich zum Umfüllen von Flüssigkeiten aller Art im medizinischen Bereich, beispielsweise zur Infusion von Flüssigkeiten in den menschlichen Körper. Dabei ist der Behälter (a) vorzugsweise ein Infusionsbeutel. Die Flüssigkeiten sind beispielsweise Blut, Blutplasma oder wässrige isotone Lösungen mit dem Elektrolytgehalt des Blutserums, welche beispielsweise Medikamente oder Nährstoffe enthalten (beispielsweise zur parenteralen Ernährung).

Ebenso bevorzugt dient die Vorrichtung zur Entnahme von Körperflüssigkeiten wie Blut, Urin oder Wundflüssigkeit.

In einer bevorzugten Ausführungsform handelt es sich bei der erfindungsgemäßen Vorrichtung um eine Vorrichtung zur Peritonealdialyse.

Die Peritonealdialyse, auch Bauchfelldialyse genannt, ist eine Variante der künstlichen Blutwäsche. Bei gesunden Menschen filtern die Nieren Stoffe aus dem Blut, damit sie mit dem Urin ausgeschieden werden können. Wenn die Nieren nicht mehr in der Lage sind, die anfallenden Stoffwechselprodukte auszuscheiden, muss das Blut künstlich gereinigt werden.

Während bei der künstlichen Niere (Hämodialyse) das Blut außerhalb des Körpers mit einem speziellen Filter gereinigt wird, benutzt man bei der Peritonealdialyse das gut durchblutete Bauchfell des Patienten als körpereigene Filtermembran. Das Bauchfell kleidet die gesamte Bauchhöhle aus. Bei der Bauchfelldialyse lässt man mehrmals am Tag eine Dialyselösung in die Bauchhöhle fließen, welche die giftigen Stoffwechselprodukte aufnimmt.

Die Peritonealdialyse führt der Patient - im Gegensatz zur Hämodialyse - alleine zu Hause durch und kann den Zeitplan nach seinen Bedürfnissen entsprechend flexibel gestalten. Bei der Peritonealdialyse sind die Patienten bezüglich der Nahrungs- und Flüssigkeitsaufnahme weniger eingeschränkt als bei der Hämodialyse. Jedoch besteht durch den permanent in der Bauchhöhle liegenden Katheter das Risiko von Infektionen an der Austrittsstelle oder in der Bauchhöhle.

Bei der Peritonealdialyse wird das Bauchfell als Blutfilter eingesetzt. Das Bauchfell (Peritoneum) ist eine gut durchblutete, halbdurchlässige Membran, welche die Bauchhöhle auskleidet und viele Organe überzieht. Über einen Katheter wird Dialyseflüssigkeit in die Bauchhöhle eingefüllt. In dieser Dialyseflüssigkeit ist eine andere Konzentration an Substanzen, als im Blut. Nach dem Prinzip der Osmose werden diese Substanzen dem Blut entzogen und gelangen in die Bauchhöhle. Nach einigen Stunden wird die Dialyseflüssigkeit mit den Harnbestandteilen wieder aus der Bauchhöhle ausgelassen.

Bei der Peritonealdialyse füllt der Patient selbst 1,5 bis 3,0 l einer sterilen Dialyselösung über einen Katheter in die Bauchhöhle, die das Bauchfell (Peritoneum) damit umspült. Die Substanzen, die ausgeschieden werden sollen, wandern vom Blut durch das Peritoneum in die Dialyselösung.

Eine weitere Aufgabe der Dialyse besteht darin, dem Körper überschüssiges Wasser zu entziehen - der Fachmann spricht von Ultrafiltration. Deshalb enthalten die meisten Dialyselösungen Glukose (Zucker). Durch einen einfachen osmotischen Vorgang wandert bei der Peritonealdialyse auch Wasser in die Dialyselösung und kann so entfernt werden. Nach etwa vier bis fünf Stunden ist die Dialyselösung, das sogenannte Dialysat, mit Giftstoffen gesättigt. Es wird aus dem Bauchraum über den Katheter abgelassen und durch frische Dialyselösung ersetzt.

Zur Durchführung der Peritonealdialyse gibt es verschiedene Möglichkeiten: Bei der kontinuierlichen ambulanten Peritonealdialyse (CAPD) wechseln die Patienten selbst etwa vier bis fünf Mal am Tag ihre Dialyselösung. Bei der automatischen Peritonealdialyse (APD) übernimmt ein Dialysegerät (Cycler) den automatischen Beutelwechsel über Nacht - so ist der Patient tagsüber noch unabhängiger und fühlt sich kaum eingeschränkt.

Die Peritonealdialyse entspricht weitgehend der natürlichen Arbeitsweise der Niere, da sie den Körper kontinuierlich und gleichmäßig entgiftet und entwässert. Der Patient muss daher generell mit weniger Nebenwirkungen rechnen. Während der Dialyse ist der Patient mobil und unabhängig und kann seiner gewohnten Tätigkeit und seinem Beruf nachgehen.

Bei den Verfahren zur kontinuierlichen ambulanten Peritonealdialyse bzw. CAPD werden im Allgemeinen zwei Behälter benötigt, um das Verfahren durchzuführen. Ein erster Behälter (Ablauf- oder Drainagebeutel) ist gemeinsam mit einem Schlauchstück vorgesehen, welches über einen Katheter an das Peritoneum eines Patienten anschließbar ist, um das Peritoneum zu entleeren. Ein zweiter Behälter (Lösungs- oder Dialysatbeutel) enthält ein Dialysat für die Zuführung zu dem Peritoneum eines Patienten. Nachdem die Flüssigkeit aus dem Peritoneum in den Ablaufbeutel abgelaufen ist, wird der Lösungsbeutel an den Patienten angeschlossen. Das Dialysat wird durch den Schlauch aus dem Lösungsbeutel an das Peritoneum des Patienten abgegeben. Darüber hinaus gibt es auch so genannte Singlebeutel-Systeme, die nur einen Lösungsbeutel und einen Verbindungsschlauch mit Patientenanschluss aufweisen. In diesem Fall wird nach erfolgter Dialyse die Lösung aus dem Peritoneum in den alten, zuvor entleerten Dialysatbeutel zurückgeführt. Dieses erfordert jedoch, das die Peritonealvorrichtung nach Entleerung in das Peritoneum für die gesamte Dauer der Dialyse am Patienten verbleibt. Singlebeutel kommen auch für die APD in Frage, wobei das Dialysegerät die verbrauchten Dialysatlösungen entsorgt.

Eine Aufgabe der vorliegenden Erfindung ist es demgemäß auch, die bekannten Vorrichtungen zur Peritonealdialyse weiterzuentwickeln, so dass Vorrichtungen erhalten werden, deren Verbindung zwischen den jeweiligen Behältern sowie dem Peritoneum des Patienten vorzugsweise kostengünstig herzustellen sind und welche vorzugsweise nicht zu einem Kinking neigen. Gleichzeitig sollte die Vorrichtung vorzugsweise eine leichte Handhabung für den Patienten ermöglichen, so dass die Gefahren einer Infektion des Peritoneums des Patienten minimiert werden.

In einer bevorzugten Ausführungsform handelt es sich daher bei der erfindungsgemäßen Vorrichtung um eine Vorrichtung zur Peritonealdialyse (1), gemäß Anspruch 4.

Es ist somit vorgesehen, dass bei bekannten Peritonealvorrichtungen die dort verwendeten Schlauchverbindungen durch Verbindungs-Folienschläuche ersetzt werden. Folienschläuche sind im Vergleich zu den bekannten Schlauchverbindungen kostengünstiger herzustellen, so dass die resultierende Vorrichtung zur Peritonealdialyse in sämtlichen Kliniken, insbesondere in armen Ländern, für den Einsatzfall ohne größere Investitionskosten auf Lager gehalten werden kann. Darüber hinaus neigen Folienschläuche nicht zum Kinking, was eine einfachere und sichere Handhabung der Vorrichtung gewährleistet.

Die Erfindung betrifft in einer besonders bevorzugten Ausführungsform eine Vorrichtung (1), umfassend ein Single- oder Doppelbeutelsystem zum Gebrauch bei der Durchführung der Peritonealdialyse, welche mindestens die folgenden Bestandteile aufweist:
(a) einen Lösungsbeutel (Dialysatbeutel) (3);
(b) einen Verbindungs-Folienschlauch (2) mit Patientenanschluss und
(c) gegebenenfalls einen Abfallbeutel (Drainagebeutel) (4).
wobei der Verbindungs-Folienschlauch (2) den Dialysatbeutel (3) über die Verbindung (5) des Folienschlauches (2) und gegebenenfalls den Drainagebeutel (4) über die gegebenenfalls vorhandene Verbindung (7) des Folienschlauches (2) miteinander verbindet und eine Verbindung (6) für einen Anschluss des Peritoneums des Patienten umfasst.

In einer weiteren bevorzugten Ausführungsform ist der Verbindungs-Folienschlauch durch Peelnähte und/oder Ventile in Kammern unterteilt.

In einer weiteren bevorzugten Ausführungsform sind je nach gewünschtem Verfahrensschritt der Peritonealdialyse die Verbindungen (5), (6) und/oder (7) verschließbar. Zum Verschließen eignen sich dabei beispielsweise trennbare Peelnähte, Ventile oder Klammern. Die Funktionen Auslauf, Einlauf und Flush werden daher vorzugsweise über trennbare Peelnähte, Ventile oder Klammern gesteuert.

Zu Beginn eines typischen Zyklus der kontinuierlichen ambulanten Peritonealdialyse wird die in der Peritonealhöhle befindliche Lösung aus der Peritonealhöhle entfernt. Falls die erfindungsgemäße Vorrichtung einen Drainagebeutel (4) umfasst, so wird die Lösung in der Peritonealhöhle über den Katheter und den daran angeschlossenen Verbindungs-Folienschlauch (2) in den vorhandenen Drainagebeutel (4) überführt. Zu diesem Zweck ist die Verbindung (5) zwischen dem Dialysatbeutel (3) und dem Folienschlauch (2) vorzugsweise geschlossen. Nach der Drainage wird dann eine Verbindung zwischen dem Dialysatbeutel (3) und der Peritonealhöhle des Patienten über den Verbindungs-Folienschlauch ermöglicht, während gleichzeitig die Verbindung zwischen Peritonealhöhle und dem Drainagebeutel (4) unterbunden wird.

Im Neuzustand des Systems, d.h. vor der Verwendung zur Peritonealdialyse, können sowohl die Beutel (3) und (4), falls vorhanden, als auch der Verbindungs-Folienschlauch (2) mit dem Dialysat gefüllt sein.

In einer weiteren bevorzugten Ausführungsform der Erfindung sind der Dialysatbeutel (3), gegebenenfalls der Drainagebeutel (4) und der Verbindungs-Folienschlauch (2) der Vorrichtung (1) integral miteinander verbunden.

Der Dialysatbeutel (3) der Vorrichtung ist für die Aufnahme des Dialysats bestimmt. Bei der Dialysatflüssigkeit kann es sich beispielsweise um eine Glucoselösung für die Peritonealdialyse handelt.

Üblicherweise hat der Dialysatbeutel ein Volumen von 500 bis 5000 ml, vorzugsweise 1000 bis 3000 ml, insbesondere 1250 bis 2750 ml. Insbesondere für die Verwendung der erfindungsgemäßen Vorrichtung bei (Klein-)Kindern können die verwendeten Dialysatbeutel auch kleiner ausgeprägt sein. Ferner kann der Dialysatbeutel (3) in mehrere, d.h. in mindestens zwei Kammern unterteilt sein, wobei zwischen den jeweiligen Kammern Ventile und/oder Peelnähte vorgesehen sind, die ein Mischen der Kompartimente vor der Anwendung für Lösungen ermöglichen, die gemischt nicht haltbar sind.

Der Dialysatbeutel (3) wird während der Peritonealdialyse an einem Stativ derart aufgehängt, dass die Dialysatflüssigkeit unter der Einwirkung des hydrostatischen Drucks in Richtung auf die durch die Aufhängung des Dialysatbeutels bedingte oben liegende Verbindung (5) des Folienschlauches abfließen kann. Daher ist an dem Dialysatbeutel (3) vorzugsweise mindestens eine Aufhängeöffnung vorgesehen, die dazu dient, mit einem Stativ in Eingriff zu gelangen, so dass die Anordnung des Dialysatbeutels (3) für die Verabreichung des Dialysats richtig orientiert ist. Der Dialysatbeutel (3) selbst kann weiterhin zusätzlich mindestens einen Stutzen aufweisen, über den Wirkstoffe und/oder Medikamente dem Dialysatbeutel (3) und damit der Dialysatflüssigkeit zugesetzt werden können.

An der oben liegenden Verbindung (5) des Verbindungs-Folienschlauches (2) ist somit der Dialysatbeutel (3) mit der Dialysatlösung angebunden. Der Verbindungs-Folienschlauch (2) ist mit dem Dialysatbeutel (3) vorzugsweise unter Einhaltung der Sterilitätsbedingungen verbunden, da bei der Peritonealdialyse die dem Peritoneum zugeführte Dialysatlösung in unsterilem Zustand ansonsten zu einer Peritonitis führen kann.

Der Verbund zwischen dem Dialysatbeutel (3) und dem Verbindungs-Folienschlauch (2) kann erfindungsgemäß dabei auf jede geeignete Weise erfolgen:

Beispielhaft ist hierfür das Verschweißen des Verbindungs-Folienschlauches (1) mit dem Dialysatbeutel (3) genannt, wobei die Außenseite des Verbindungs-Folienschlauches mit der Innenseite des Dialysatbeutels oder aber die Innenseite des Verbindungs-Folienschlauchs mit dem Beutel verschweißt ist. Der Verbindungs-Folienschlauch ist dabei über Peelnähte oder Ventile abgetrennt.

Alternativ kann der Dialysatbeutel (3) auch über ein oben beschriebenes Portelement mit dem Folienschlauch verbunden werden. In diesem Fall kann das Portelement mit dem Beutel und dem Verbindungs-Folienschlauch als Zwischenglied verschweißt sein.

Damit die Dialysatflüssigkeit nicht unmittelbar nach dem Anschluss der Vorrichtung an den Patienten in dessen Peritoneum fließt, kann die Verbindung (5) zwischen Drainagebeutel (3) und Folienschlauch (2) zunächst verschlossen sein. Hierfür eignet sich neben den bereits genannten Verschlussarten auch eine Aufreißversiegelung, wie eine Peelnaht.

Der Verbindungs-Folienschlauch (2) besitzt eine zweite Verbindung (6) zum Peritoneum eines Patienten. Diese zweite Verbindung (6) wird vorzugsweise mit einem Katheder verbunden, der wiederum in das Peritoneum des Patienten führt. Bei dem Katheter handelt es sich im Allgemeinen um einen Dauerkatheter. Die Verbindung (6) des erfindungsgemäß vorgesehenen Folienschlauches (2) für den Katheder ist vorzugsweise als ein Y-Teil oder ein Sattel ausgebildet, der auf den Folienschlauch aufgebracht ist. Der Sattel kann dabei beispielsweise wie in der WO 90/06262 beschrieben ausgebildet sein.

Das auf dem Verbindungs-Folienschlauch zum Anschluss an den Katheter angebrachte Y-Teil bzw. der auf dem Verbindungs-Folienschlauch zum Anschluss an den Katheter angebrachte Sattel sollte vorzugsweise folgende Strömungssequenzen ermöglichen:
(1) Die Drainagelösung sollte aus der Bauchhöhle des Patienten in den gegebenenfalls vorhandenen leeren Drainagebeutel auf dem Boden fließen können.
(2) Die Dialysatflüssigkeit sollte durch den Folienschlauch, gegebenenfalls den Sattel bzw. gegebenenfalls das Y-Teil in den gegebenenfalls vorhandenen Drainagebeutel hindurchgespült werden, um die Leitung zu reinigen.
(3) Die Dialysatflüssigkeit sollte in die Bauchhöhle des Patienten eingeführt werden.

Der Sattel bzw. das Y-Teil sollte darüber hinaus vorzugsweise dafür sorgen, dass keine Flüssigkeit in unnötiger Weise verloren geht, nachdem die Vorrichtung vom Patienten getrennt wurde.

Der Verbindungs-Folienschlauch (2) besitzt darüber hinaus gegebenenfalls eine dritte Verbindung (7), die an einen Drainagebeutel (4) angebunden sein kann.

Üblicherweise hat dieser Drainagebeutel (4) ein gleiches oder größeres Volumen als der Dialysatbehälter, da insbesondere bei der Verwendung von Glucose-haltigen Dialysatlösungen Wasser bei der Peritonealdialyse aus dem Körper in das Dialysat und damit anschließend in den Drainagebeutel (4) überführt wird.

In einer bevorzugten Ausführungsform ist das Volumen des Drainagebeutels (4) 1,5fach größer als das Volumen des Dialysatbeutels (3)

Der Verbindungs-Folienschlauch (2) ist mit dem Drainagebeutel (4) vorzugsweise unter Einhaltung der Sterilitätsbedingungen verbunden.

Der Verbund zwischen dem Drainagebeutel (4) und dem Verbindungs-Folienschlauch (2) kann dabei auf jede geeignete Weise erfolgen:

Beispielhaft ist hierfür das Verschweißen des Folienschlauches (1) mit dem Drainagebeutel (4) genannt, wobei - je nach verwendetem Material des Drainagebeutels (4) und des Folienschlauches (2) - der Verbindungs-Folienschlauch (2) entweder mit seiner Außenseite oder seiner Innenseite des Drainagebeutels (4) verschweißt ist. Zur Trennung zum Lösungszweig ist vorzugsweise eine Peelnaht, ein Ventil oder eine Klemme vorgesehen.

Alternativ kann der Drainagebeutel (4) auch über ein Portelement, wie beispielsweise ein Sattel, mit dem Folienschlauch verbunden werden. In diesem Fall kann das Portelement mit dem Beutel und dem Verbindungs-Folienschlauch als Zwischenglied verschweißt sein. Bei einer Ausführungsform der vorliegenden Erfindung sind der Behälter für die Dialysatflüssigkeit (3) und der Drainagebeutel (4) aus biegsamen Kunststoffmaterialen hergestellt. Dabei handelt es sich bevorzugt um coextrudierte Mehrschichtfolien aus Kunststoffen, die auf gegebenenfalls modifizierten Polyolefinen basieren.

In einer bevorzugten Ausführungsform der vorliegenden Erfindung sind die für die erfindungsgemäße Vorrichtung verwendeten Behälter (3) und (4), falls vorhanden, frei von PVC.

In einer weiteren bevorzugten Ausführungsform der vorliegenden Erfindung sind die für die erfindungsgemäße Vorrichtung verwendeten Behälter (3) und (4), falls vorhanden, frei von Weichmachern.

In einer weiteren bevorzugten Ausführungsform der vorliegenden Erfindung sind die für die erfindungsgemäße Vorrichtung verwendeten Behälter (3) und (4), falls vorhanden, frei von PVC und Weichmachern.

Eine bevorzugte Ausführungsform der erfindungsgemäßen Vorrichtung dient zur Herstellung einer Fest-Flüssig-(Powder-Liquid)-Mischung. Dabei ist der Behälter (a), der eine Flüssigkeit enthält, vorzugsweise über eine Peelnaht mit einem weiteren Behälter, vorzugsweise Beutel, verbunden, der ein Pulver enthält.

In diesem Fall erfolgt dann vor der Entnahme durch den Folienschlauch eine Vermischung von Flüssigkeit und Feststoff.

Ebenso bevorzugt können Pulver- und Flüssigkeitsbehälter über einen Folienschlauch verbunden sein, so dass die Flüssigkeit durch den Folienschlauch in den Behälter mit dem Pulver geleitet wird. Die Entnahme der Fest-Flüssig-Mischung kann dann über eine weitere Konnektionsstelle, die an einem der Behälter oder dem Folienschlauch angebracht ist, erfolgen.

Die bevorzugte Ausführungsform der erfindungsgemäßen Vorrichtung bietet den Vorteil, dass Feststoff und Flüssigkeit getrennt unter sterilen Bedingungen (für den Feststoff beispielsweise durch Bestrahlen) verpackt werden können und erst unmittelbar vor Gebrauch in Kontakt kommen.

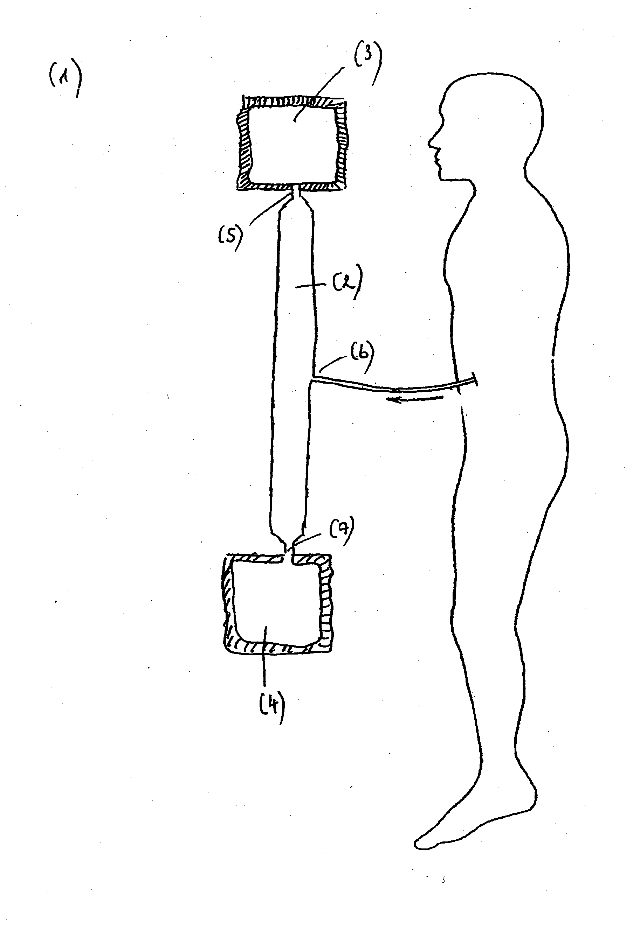

## Patentansprüche

1. Medizinische Vorrichtung, umfassend
(a) einen zur Aufnahme von Flüssigkeiten geeigneten Behälter;
(b) damit verbunden ein Folienschlauch mit einer Konnektionsstelle und
(c) gegebenenfalls einen oder mehrere weitere, mit dem Folienschlauch verbundene, zur Aufnahme von Flüssigkeiten geeignete Behälter,
**dadurch gekennzeichnet, dass** der Verbindungs-Folienschlauch durch Peelnähte und/oder Ventile in Kammern unterteilt ist.

2. Vorrichtung gemäß Anspruch 1, **dadurch gekennzeichnet, dass** die Innenfläche des Folienschlauchs strukturiert ist.

3. Vorrichtung gemäß Anspruch 2, **dadurch gekennzeichnet, dass** die Innenfläche des Folienschlauchs durch Aufrauhen strukturiert ist und eine Rauhtiefe R₂ gemäß DIN/DIS 4287/1 von 0,05 bis 10 µm aufweist.

4. Vorrichtung (1) gemäß einem der Ansprüche 1 bis 3 zur Peritonealdialyse, **dadurch gekennzeichnet, dass**
(a) der zur Aufnahme von Flüssigkeiten geeignete Behälter ein Dialysatbeutel (3) ist;
(b) der Folienschlauch mit einer Konnektionsstelle ein Verbindungs-Folienschlauch (2) mit Patientenanschluss ist; und
(c) der weitere zur Aufnahme von Flüssigkeiten geeignete Behälter, ein Drainagebeutel (4) ist,
**dadurch gekennzeichnet, dass** der Folienschlauch (2) erste (5), zweite (6) und dritte (7) Verbindungen aufweist, wobei die erste Verbindung (5) zwischen dem Folienschlauch (2) und dem Dialysatbeutel (3) vorgesehen ist, das Peritoneum eines Patienten über die zweite Verbindung (6) an den Folienschlauch (2) angebunden werden kann und die dritte Verbindung (7) zwischen dem Drainagebeutel (4) und dem Folienschlauch (2) vorgesehen ist.

5. Vorrichtung gemäß einem der Ansprüche 1 bis 3, **dadurch gekennzeichnet, dass** der Folienschlauch eine Wandstärke von 50-120 µm aufweist.

6. Vorrichtung nach einem der Ansprüche 1 bis 5, **dadurch gekennzeichnet, dass** der Behälter (a), gegebenenfalls der Behälter (c) und der Verbindungs-Folienschlauch der Vorrichtung integral miteinander verbunden sind.

7. Vorrichtung nach einem der Ansprüche 1 bis 6, **dadurch gekennzeichnet, dass** der Behälter (a) in mindestens zwei Kammern unterteilt ist, wobei zwischen den jeweiligen Kammern Peelnähte und/oder Ventile vorgesehen sind, die ein Verbinden oder Trennen der Kammern ermöglichen.

8. Vorrichtung nach einem der Ansprüche 1 bis 7, **dadurch gekennzeichnet, dass** der Behälter (a) zusätzlich mindestens einen Stutzen aufweist, über den Wirkstoffe und/oder Medikamente dem Behälter (a) zugesetzt werden können.

9. Vorrichtung nach einem der Ansprüche 1 bis 8, **dadurch gekennzeichnet, dass** der gegebenenfalls vorhandene Behälter (d) zusätzlich mindestens einen Stutzen aufweist, über den Teile einer Flüssigkeit entnommen werden können.

10. Vorrichtung nach einem der Ansprüche 1 bis 9, **dadurch gekennzeichnet, dass** zur Verbindung Portelemente verwendet werden.

11. Vorrichtung nach einem der Ansprüche 1 bis 10, **dadurch gekennzeichnet, dass** der Verbindungs-Folienschlauch bis zu 7 Folienschichten aufweist.

12. Vorrichtung nach einem der Ansprüche 1 bis 11, **dadurch gekennzeichnet, dass** die Folienschichten Copolymere aus Polyethylen und Polypropylen oder modifiziertes Polypropylen umfassen, wobei das Polypropylen in Mischung mit synthetischen Kautschuken, insbesondere Styrol/Ethylen-Butylen/Styrol-, Styrol/Ethylen-Propylen/Styrol- und Styrol/Isopren/Styrol-Kautschuken, vorliegt.

13. Vorrichtung nach einem der Ansprüche 1 bis 12, **dadurch gekennzeichnet, dass** der Verbindungs-Folienschlauch mindestens 2 Schichten umfasst, wobei
- der Hauptbestandteil der äußeren Schicht ein Homopolymer aus Polypropylen ist, dessen Schmelzpunkt ungefähr bei 161 °C liegt,
- der Hauptbestandteil der inneren Schicht ein Polypropylen-Polymer ist, dessen Schmelzpunkt größer 135 °C ist.

14. Vorrichtung nach einem der Ansprüche 1 bis 13, **dadurch gekennzeichnet, dass** die Außenseite des Verbindungs-Folienschlauchs (b) mit der Innenseite des Behälters (a) verschweißt ist.

15. Vorrichtung nach einem der Ansprüche 1 bis 3 oder 5 bis 14, wobei der Behälter (a) zur Aufnahme von Flüssigkeit über eine Peelnaht oder durch den Folienschlauch (b) mit einem weiteren Behälter verbunden ist, der eine Kammer zur Aufnahme eines Pulvers enthält,

16. Verfahren zur Herstellung einer Vorrichtung nach einem der Ansprüche 1 bis 15, **gekennzeichnet durch** die folgenden Verfahrensschritte:
(1) Bereitstellen der Vorrichtungskomponenten a), b) und c) gemäß Anspruch 1 als gegebenenfalls randbeschnittene Schlauchfolie in Form von Rollenwaren und gegebenenfalls von Portelementen als Einzelteile;
(2) Erzeugen der Beutelkontur oder der Beutelkonturen **durch** Erhitzen unter Druck mittels Schweißvorrichtungen;
(3) Einführen des Verbindungs-Folienschlauches oder des Portelements in die Beutelkontur(en) und Verschweißen mit der/den Beutelkontur(en); gegebenenfalls Verschweißen des Portelements mit dem Verbindungs-Folienschlauch.
(4) Anbringen des Patientenanschlusses in oder auf dem Verbindungs-Folienschlauch **durch** Verschweißen,
wobei der Verbindungs-Folienschlauch **durch** Peelnähte und/oder Ventile in Kammern unterteilt ist.

## Claims

1. Medical device comprising:
(a) a container suitable for holding liquids;
(b) connected thereto a film tube with a connection site, and
(c) optionally, one or more further containers connected to the film tube and suitable for holding liquids,
**characterized in that** the connecting film tube is divided into chambers by peel seams and/or valves.

2. Device according to Claim 1, **characterized in that** the inner surface of the film tube is structured.

3. Device according to Claim 2, **characterized in that** the inner surface of the film tube is structured by roughening and has a surface roughness R2 of 0.05 to 10 µm according to DIN/DIS 4287/1.

4. Device (1) according to one of Claims 1 to 3 for peritoneal dialysis, **characterized in that**
(a) the container suitable for holding liquids is a dialysis bag (3);
(b) the film tube with a connection site is a connecting film tube (2) with a patient attachment; and
(c) the further container suitable for holding liquids is a drainage bag (4),
**characterized in that** the film tube (2) has first (5), second (6) and third (7) connections, the first connection (5) being provided between the film tube (2) and the dialysate bag (3), the peritoneum of a patient being able to be linked to the film tube (2) via the second connection (6), and the third connection (7) being provided between the drainage bag (4) and the film tube (2).

5. Device according to one of Claims 1 to 3, **characterized in that** the film tube has a wall thickness of 50-120 µm.

6. Device according to one of Claims 1 to 5, **characterized in that** the container (a), optionally the container (c), and the connecting film tube of the device are integrally connected to each other.

7. Device according to one of Claims 1 to 6, **characterized in that** the container (a) is divided into at least two chambers, wherein peel seams and/or valves are provided between the respective chambers and permit connection or separation of the chambers.

8. Device according to one of Claims 1 to 7, **characterized in that** the container (a) additionally has at least one nozzle through which active substances and/or medicaments can be added to the container (a).

9. Device according to one of Claims 1 to 8, **characterized in that** the optionally present container (c) additionally has at least one nozzle through which some liquid can be removed.

10. Device according to one of Claims 1 to 9, **characterized in that** port elements are used for the connection.

11. Device according to one of Claims 1 to 10, **characterized in that** the connecting film tube has up to 7 film layers.

12. Device according to one of Claims 1 to 11, **characterized in that** the film layers comprise copolymers of polyethylene and polypropylene or modified polypropylene, the polypropylene being present in mixture with synthetic rubbers, in particular with styrene/ethylene-butylene/styrene, styrene/ethylene-propylene/styrene, and styrene/ isoprene/styrene rubbers.

13. Device according to one of Claims 1 to 12, **characterized in that** the connecting film tube comprises at least 2 layers, wherein
- the main constituent of the outer layer is a homopolymer of polypropylene, of which the melting point is approximately 161°C,
- the main constituent of the inner layer is a polypropylene polymer, of which the melting point is higher than 135°C.

14. Device according to one of Claims 1 to 13, **characterized in that** the outer side of the connecting film tube (b) is welded to the inner side of the container (a).

15. Device according to one of Claims 1 to 3 or 5 to 14, wherein the container (a) for holding liquid is connected by a peel seam or by the film tube (b) to a further container, which comprises a chamber for holding a powder.

16. Method for producing a device according to one of Claims 1 to 15, **characterized by** the following method steps:
(1) provision of the device components a), b) and c) according to Claim 1 as an optionally edge-trimmed film tube in the form of rolled articles, and optionally of port elements as individual parts;
(2) production of the bag contour or bag contours by heating at pressure using welding devices;
(3) insertion of the connecting film tube or of the port element into the bag contour(s) and welding to the bag contour(s), optionally welding the port element to the connecting film tube;
(4) fitting the patient attachment in or on the connecting film tube by welding,
wherein the connecting film tube is divided into chambers by peel seams and/or valves.

## Revendications

1. Dispositif médical, comprenant
(a) un contenant destiné à recevoir des liquides ;
(b) un tuyau en feuille mince à paroi très déformable, relié à ce dernier et muni d'une zone de raccordement ; et
(c) le cas échéant, un ou plusieurs contenants supplémentaires destinés à recevoir des liquides et reliés au tuyau en feuille mince à paroi très déformable,
**caractérisé en ce que** ledit tuyau de liaison en feuille mince à paroi très déformable est divisé en chambres par des joints pelables et/ou des valves.

2. Dispositif selon la revendication 1, **caractérisé en ce que** la surface intérieure du tuyau en feuille mince à paroi très déformable est structurée.

3. Dispositif selon la revendication 2, **caractérisé en ce que** la surface intérieure du tuyau en feuille mince à paroi très déformable est structurée par la formation de stries et possède une profondeur de rugosité R2 selon DIN/DIS 4287/1 de 0,05 à 10 µm.

4. Dispositif (1) selon l'une quelconque des revendications 1 à 3 destiné à la dialyse péritonéale, **caractérisé en ce que**
(a) le contenant destiné à recevoir des liquides est une poche de dialysat (3) ;
(b) le tuyau en feuille mince à paroi très déformable muni d'une zone de raccordement est un tuyau de liaison en feuille mince à paroi très déformable (2) avec une jonction vers le patient ; et
(c) le contenant supplémentaire destiné à recevoir des liquides est une poche de drainage (4),
**caractérisé en ce que** le tuyau en feuille mince à paroi très déformable (2) comporte un premier (5), un deuxième (6) et un troisième (7) raccord, ledit premier raccord (5) étant prévu entre le tuyau en feuille mince à paroi très déformable (2) et la poche de dialysat (3), le péritoine du patient pouvant être relié au tuyau en feuille mince à paroi très déformable (2) par l'intermédiaire du deuxième raccord (6), et le troisième raccord (7) étant prévu entre la poche de drainage (4) et le tuyau en feuille mince à paroi très déformable (2).

5. Dispositif selon l'une quelconque des revendications 1 à 3, **caractérisé en ce que** le tuyau en feuille mince à paroi très déformable a une épaisseur de paroi de 50 à 120 µm.

6. Dispositif selon l'une quelconque des revendications 1 à 5, **caractérisé en ce que** le contenant (a), le cas échéant le contenant (c) et le tuyau de liaison en feuille mince à paroi très déformable du dispositif sont intégralement reliés entre eux.

7. Dispositif selon l'une quelconque des revendications 1 à 6, **caractérisé en ce que** le contenant (a) est divisé en au moins deux chambres, des soudures de faible résistance et/ou des valves, qui permettent de relier ou de séparer lesdites chambres, étant prévues entre lesdites chambres.

8. Dispositif selon l'une quelconque des revendications 1 à 7, **caractérisé en ce que** le contenant (a) comporte en plus au moins une tubulure, via laquelle des agents actifs et/ou des médicaments peuvent être ajoutés dans le contenant (a).

9. Dispositif selon l'une quelconque des revendications 1 à 8, **caractérisé en ce que** le contenant (d) éventuellement présent comporte en plus au moins une tubulure, via laquelle il est possible de prélever des parties d'un liquide.

10. Dispositif selon l'une quelconque des revendications 1 à 9, **caractérisé en ce que** des éléments d'interface sont utilisés pour la liaison.

11. Dispositif selon l'une quelconque des revendications 1 à 10, **caractérisé en ce que** le tuyau de liaison en feuille mince à paroi très déformable comporte jusqu'à 7 couches membranaires.

12. Dispositif selon l'une quelconque des revendications 1 à 11, **caractérisé en ce que** les couches membranaires comportent des copolymères en polyéthylène et polypropylène ou polypropylène modifié, le polypropylène étant présent sous forme de mélange avec des caoutchoucs synthétiques, en particulier des caoutchoucs de styrène/éthylène-butylène/styrène, des caoutchoucs de styrène/éthylène-propylène/styrène et des caoutchoucs de styrène/isoprène/styrène.

13. Dispositif selon l'une quelconque des revendications 1 à 12, **caractérisé en ce que** le tuyau de liaison en feuille mince à paroi très déformable comporte au moins deux couches,
- le composant principal de la couche extérieure étant un homopolymère en polypropylène, dont le point de fusion se situe à peu près à 161°C,
- le composant principal de la couche intérieure étant un polymère polypropylène, dont le point de fusion est supérieur à 135°C.

14. Dispositif selon l'une quelconque des revendications 1 à 13, **caractérisé en ce que** la face extérieure du tuyau de liaison en feuille mince à paroi très déformable (b) est soudée à la face intérieure du contenant (a).

15. Dispositif selon l'une quelconque des revendications 1 à 3 ou 5 à 14, dans lequel le contenant (a) destiné à recevoir un liquide est relié, par l'intermédiaire d'un joint pelable ou par l'intermédiaire du tuyau en feuille mince à paroi très déformable (b), à un autre contenant qui contient une chambre destinée à recevoir une poudre.

16. Procédé permettant de réaliser un dispositif selon l'une quelconque des revendications 1 à 15, **caractérisé par** les étapes suivantes :
(1) mise à disposition des composants a), b) et c) selon la revendication 1 pour le dispositif, sous la forme d'un tuyau en feuille mince à paroi très déformable, éventuellement à bords découpés, en forme de matériau en rouleau et, éventuellement, d'éléments d'interface sous forme de pièces détachées ;
(2) réalisation du contour de poche ou des contours de poche moyennant un chauffage sous pression avec des dispositifs de soudage ;
(3) introduction du tuyau de liaison en feuille mince à paroi très déformable ou de l'élément d'interface dans le/les contour(s) de poche et soudage au/aux contour(s) de poche ; le cas échéant, soudage de l'élément d'interface au tuyau de liaison en feuille mince à paroi très déformable ;
(4) pose de la jonction avec le patient dans ou sur le tuyau de liaison en feuille mince à paroi très déformable moyennant un soudage,
ledit tuyau de liaison en feuille mince à paroi très déformable étant divisé en chambres par l'intermédiaire de joints pelables et/ou de valves.
